# EUROPEAN PATENT SPECIFICATION

(11) **EP 2 637 552 B1**
(45) Date of publication and mention of the grant of the patent: **20.03.2019**
(21) Application number: 11782477.1
(22) Date of filing: 24.10.2011
(51) Int. Cl.: A61B 5/00, A61B 5/11

(54) **LOCATION BASED WIRELESS MEDICAL DEVICE**
STANDORTBASIERTE DRAHTLOSE MEDIZINISCHE VORRICHTUNG
DISPOSITIF MÉDICAL SANS FIL GÉODÉPENDANT

(30) Priority: 08.11.2010 US 410992 P
(43) Date of publication of application: 18.09.2013
(73) Proprietor: Koninklijke Philips N.V., 5656 AE Eindhoven (NL)
(72) Inventor: PATEL, Maulin, Dahyabhai, 5656 AE Eindhoven (NL)
(74) Representative: Steffen, Thomas
(86) International application number: PCT/IB2011/054732
(87) International publication number: WO 2012/063154

(56) References cited:
- GB-A- 2 425 601
- US-A1- 2005 137 573
- US-A1- 2009 048 492
- US-A1- 2009 063 187

## Description

The present application relates to wireless devices. In particular, it relates to wireless sensor networks, such as body area networks (BANs) or patient area networks (PANs), which monitor a patient's physiological parameter and transmit a data regarding the sensed parameters to a control system.

Patients have traditionally been monitored using sensing units connected by wires to a base station. These wires inhibited the patient mobility and were labor intensive to install. To improve patient mobility, facilitate installation, and eliminate wire clutter, wireless sensing units have been developed. Certain patients require continuous monitoring of physiological parameters, such as ECG, SpO₂, blood pressure, blood sugar, or the like. Though well enough to move about the community, they were restricted to a hospital room, the hospital ward, a convalescent room, or their home to facilitate continuous monitoring of physiological parameters. To venture out of these areas, the patients would be unmonitored.

In order to continuously monitor patient physiological parameters without constraining their activities, it is desirable to be able to mount sensors on the body of the patient, which are light and compact as possible while also being capable of communicating wirelessly with each other and a base station. A body area network (BAN) includes multiple nodes which are typically sensors that can be either wearable or implantable in to the human body. The nodes monitor vital body parameters and/or movements, and communicate with each other over a wireless medium. The nodes can transmit physiological data from a body to a control unit from which the data can be forwarded, in real-time, to a hospital, clinic, or elsewhere over a local area network (LAN), wide area network (WAN), a cellular network, or the like.

Wireless technology provides convenient and unobtrusive connectivity between these devices. With the advances in energy efficient, reliable, low cost and high rate wireless technology, a variety of wireless consumer electronic devices have become integral part of our day to day life. Due to the portable nature of many of these devices, the likelihood of their proliferation beyond the regions in which they are authorized to operate has increased significantly. In particular, the wireless medical sensor devices designed to operate, in, on and around the human body to monitor and control various physiological parameters are expected to be carried globally by humans. Average human beings are unlikely to be aware of complex regulations governing the use of wireless devices. This means that the portable wireless devices must have provisions to reconfigure themselves to comply with local regulations.

The requirements for designing wireless BANs include providing convenient and unobtrusive connectivity between the nodes while maintaining energy efficient, reliable, low cost, high rate wireless connectivity and while adhering to geopolitical regulatory requirements regarding the use of the radio spectrum. The regulatory requirements mandate the compliance with technical requirements such as frequency-band usage, duty cycle limitations, bandwidth, maximum transmit power limitations, specific absorption rate, etc. However, these regulatory requirements are not harmonized worldwide. For example, 433.05-434.79 MHz band is designated as a license-free Industrial Scientific and Medical (ISM) band in Europe but not in US. On the other hand 902-928 MHz band is designated as a license-free ISM band in US but not in Europe. Therefore, wireless devices authorized to operate in one region may not be legally authorized to operate in another region. Even if the spectrum used by the device is available worldwide, the transmit power, duty cycle and other restrictions may be different in different regions thereby inhibiting the free movement of wireless devices across the border.

Due to the portable nature of many of these devices and the expected integration into day to day life, the likelihood that the wireless BAN devices being carried globally beyond the region in which they are authorized to operate should be planned for while understanding that the average patient is unlikely to be aware of local regulations governing the use of the wireless devices in that region. This poses a severe risk for the patient because non-compliant, unauthorized use of the wireless device can be detrimental to the wireless transmission functions between the devices which can interfere with sensing and/or therapeutic functions. There exists a need for wireless BANs and wireless portable devices to adjust wireless transmission parameters to meet local regulatory requirements for wireless transmission.

The present application provides a new and improved method and system for location based wireless patient monitoring and therapy delivery which overcomes the above-referenced problems and others.

In US 2009/0063187 A1 a portable source medical device is presented. The device determines communication links of a network presently available to effect communications with a target component when the source medical device is at each of a multiplicity of geographical locations. A profile is generated comprising information about each available communication link and attributes associated with each available communication link for each geographical location. When the source medical device is at a particular geographical location, a profile associated with the particular geographical location is accessed and a network connection is established between the source medical device and the target.

In GB 2 425 601 A an apparatus for monitoring and/or treating a substance or body is presented. The apparatus comprises a sensor and a computer device. The sensor and the computer device have radio frequency circuitry for facilitating the transmission of data between them via a wireless communication link. The computer device has means for selecting wireless link transmission properties based upon user location, and for signaling these properties to the sensor.

In US 2009/0048492 A1 a method for associating a plurality of patient monitoring sensors with an appropriate patient is disclosed. The method includes estimating a sensor location for each of a plurality of sensors that are operatively connected to a patient, transmitting the sensor location estimate from each of the sensors to a wireless device such that the sensor location estimate does not pass through an on-patient hub before reaching the wireless device, and implementing the sensor location estimate to assign each of the sensors that are disposed within a predefined region to a single patient.

In accordance with one aspect, a wireless medical device is presented. The wireless medical device includes at least one of a sensor configured to monitor physiological data of a patient and an actuator configured to deliver therapy to the patient. A wireless transceiver, which has a plurality of selectable operating parameters, is configured to transmit and/or receive information packets related to at least one of the monitored physiological data
and delivered therapy. A location management module is configured to ascertain a current geographical position of the wireless medical device and to determine a corresponding geographical region associated with the current geographical position. The location management module is configured to control the wireless transceiver to operate according to one of a plurality of operating profiles defining a plurality of operating parameters for the wireless transceiver associated with regulatory requirements of the determined geographical region. The location management module is configured to control the wireless transceiver to transmit the determined geographical region to a neighboring wireless medical device.

In accordance with another aspect, a method for wirelessly transmitting medical information is presented. The method includes at least one of monitoring physiological data of a patient and delivering therapy to the patient. Information packets related to at least one of the monitored physiological data and delivered therapy are wirelessly transmitted and/or received via a wireless transceiver. A current geographical position of a wireless medical device is ascertained and a corresponding geographical region associated with the current geographical position is determined. The wireless transceiver is controlled to operate according to one of a plurality of operating profiles defining a plurality of operating parameters for the wireless transceiver associated with regulatory requirements of the determined geographical region. The wireless transceiver is controlled to transmit the determined geographical region to a neighboring wireless medical device.

One advantage is that wireless medical devices maintain compliance to local regulatory requirements for wireless transmissions regardless of geographical location.

Still further advantages of the present invention will be appreciated by those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 is a diagrammatic illustration of a medical wireless network;
FIGURE 2 is a detailed illustration of one of the wireless medical devices of FIGURE 1;
FIGURE 3 is a diagrammatic illustration of the hub medical device of FIGURE 1; and,
FIGURE 4 is a flow chart illustrative of a method of operation.

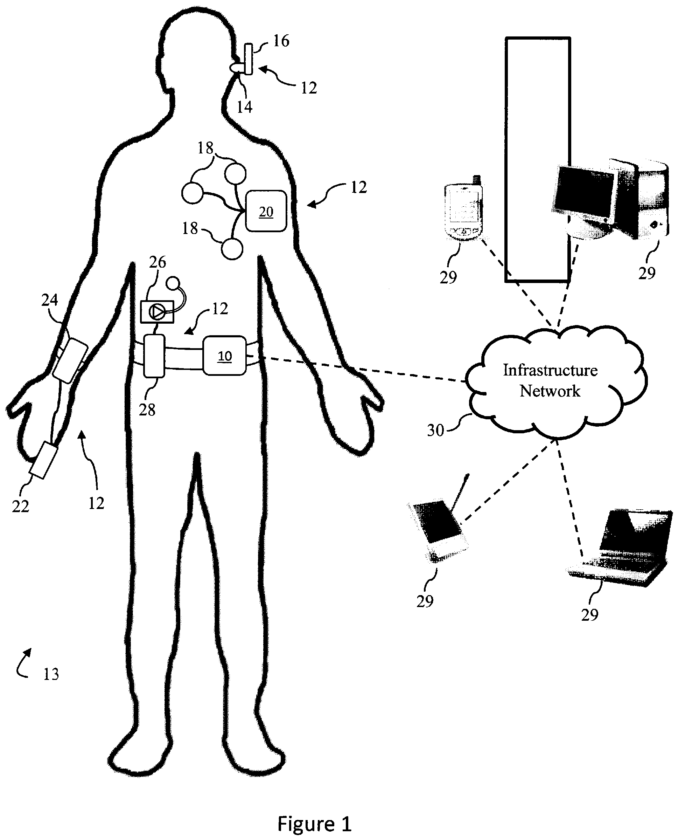

With reference to FIGURE 1, a plurality of wireless medical devices includes a hub medical device **10** and a plurality of other wireless medical devices **12,** which form a personal area network (PAN) or a body area network (BAN) **13,** arranged approximate to a patient's body for monitoring and recording various physiological parameters, administering therapy, or the like. The wireless medical devices **12** communicate wirelessly to the hub medical device **10.** Various wireless medical devices **12** are contemplated, such as an inner-ear sensor **14** connected to an associated electronic module **16** which is disposed at least partially in the patient's ear to measure temperature, blood pressure, pulse rate, or the like. As another example, the wireless medical devices **12** can include an ECG monitor having a plurality of ECG sensors or electrodes **18** connected to an electronic module **20** which measures and interprets the sensed signals. As another example, an SpO2 sensor **22** senses blood oxygen and pulse rate, which are communicated by an associated electronics module **24.** As another example, an infusion pump or other actuator **26** injects or otherwise dispenses medications into the patient's body under the control of electrical signals from an associated electrical module **28.** Other wireless medical devices **12** which sense physiological parameters or deliver therapy includes pacemakers, hearing aids, vision aids, prosthetic limbs, artificial organs, and the like.

The wireless hub **10** conveys the received signals from the wireless medical devices **12** to other wireless medical devices **29,** such as computer workstations, cellular phones, personal digital assistants, tablet computers, and the like, via an infrastructure network **30.** It should be appreciated that the hub device can be a dedicated hub for the wireless medical device **12** or a multifunction device such as a cellular phone, personal digital assistant, tablet computer, and the like. Communications between the hub and the wireless network **30** can be via a wireless local area network (LAN) based on the IEEE 802.11 standards, via a wireless wide area network (WAN) such as a cellular network, via a campus area network (CAN), via a metropolitan area networks (MAN), via relatively high power RF transmissions, or the like.

The wireless medical devices **12** and the hub **10** may interact with one another in various configurations. For example, in a star network, each of the wireless medical devices **12** communicates directly with the hub medical device **10.** The hub device receives acknowledgment signals or beacon signals from the devices **12** to, for example, synchronize the devices in anticipation of sending and receiving information packets, control signals, and the like, from the hub **10.** In a mesh network, the devices **12** communicate directly with each other and the hub **10.** Some of the devices **12** may communicate directly with the hub **10** or they may communicate with the hub **10** via other devices, such as computer, PDA's, mobile phone, or the like. These other devices may also communicate with other wireless medical devices **29** directly or via the infrastructure network **30** rather than via the hub **10.**

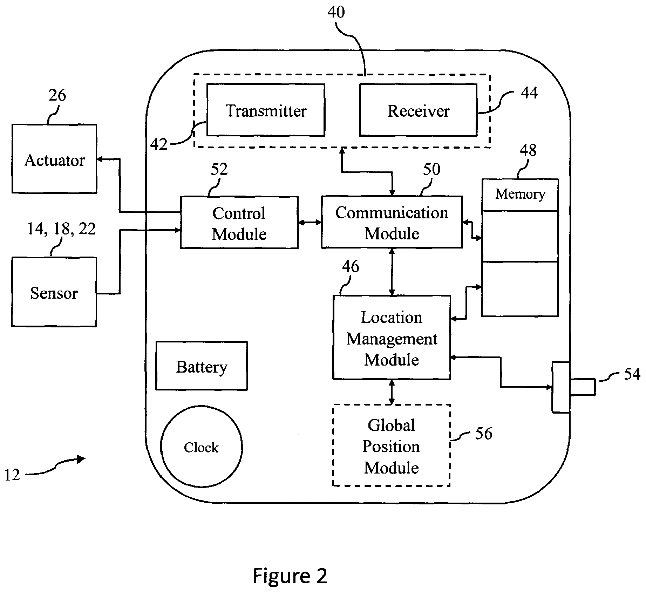

With reference to FIGURE 2, each wireless medical device **12** includes at least one of a sensor **14, 18, 22,** which monitors physiological data of the patient or an actuator **26** which delivers therapy to the patient. The electronics module **20, 24, 28,** associated with each sensor, actuator, or combination, includes a wireless transceiver **40** with a transmitter **42** and a receiver **44** which transmit and receive, respectively, information packets related to at least one of the monitored physiological data and/or the delivered therapy to/from at least one of the neighboring wireless medical device **12** and the wireless hub **10.** Each wireless transceiver has as plurality of selectable operating parameters, such as frequency, duty cycle, bandwidth, maximum transmit power, and the like.

Each wireless medical device **12** includes a location management module **46.** The location management module **46** ascertains a current geographical position of the wireless medical device **12** and determines a geographical region associated with the current geographical position. Tracking the geographical region, e.g. North America, Europe, Asia, South America, etc., of the wireless medical device ensures that the operation of transceiver **40** complies with the local regulatory requirements for wireless transmission for that region. Each geographical region is associated with at least one operating profile. Each operating profile defines a plurality of operating parameters for the wireless transceiver **40** which are associated with the geographical region. For example, an operating profile is defined for the United States of America (USA) which defines a transmission frequency, duty cycle, bandwidth and maximum transmit power for the transmitter **42** as mandated by the Federal Communications Commision (FCC). It should be appreciated that multiple operating profiles for a single geographical region are contemplated. Conversely, a single operating profile maybe associated with multiple geographical regions.

Once the geographical region is determined, the location management module **46** controls the wireless transceiver **40** to operate according to one of the operating profiles based on the determined geographical region. In one embodiment, the operating profiles are stored in a profile memory **48** of the wireless medical device **12.** In another embodiment, the operating profiles are stored remotely and accessed wirelessly or received wirelessly by the transceiver **40.** The operating profiles may be stored in a memory unit of the hub device **10.** The hub device may transmit the operating profile or the wireless medical device **12** may request the appropriate operating profile. Alternatively, the hub device 10 and/or wireless medical device **12** may wirelessly access the stored operating profiles via the infrastructure network **30.** In this arrangement, the operating profiles are stored on a computer readable medium that is part of a computer workstation or server which is part of a LAN, WAN, CAN, MAN, or the like.

A communication module **50** receives physiological information sensed by the sensor **14, 18, 22** via a sensor or actuator control module **52.** The control module **52** also communicates with the actuator **26 to** control its operation in accordance with received information packets. The communication module packages the sensed information and other transmission information such as acknowledgments, and the like, into information packets. The communication module controls the transceiver **40** to transmit the packets with an operating profile dictated by the location management module **46.** It should be appreciated that the wireless medical device may include multiple transmitters **42** as part of the transceiver **40.** Operating constraints may limit a single transmitter from operating at widely distinct frequencies. For example, a single transmitter may be capable of operating at the proposed 2.36 GHz MBAN frequency and the license free 2.4 GHz frequency. However, a second transmitter may be required to operate at the license free 433.05-434.79 MHz Industrial Scientific and Medical (ISM) band in Europe.

In one embodiment, each wireless medical devices **12** includes a user input **54,** such as a switch, button, touch pad, input device, or the like, which is operated to input the corresponding geographical region to the location management module **46.** As a switch, the user input **54** includes a plurality of user selectable position each of which is associated with at least one geographical region. As a button, the user may cycle through button presses of the user input **54** to select a corresponding geographical region or the user may select one of a plurality of buttons, each being associated with at least one geographical region. It should be appreciated that other user inputs **54,** such as joystick, keypad, keyboard, touch-screen, touchpad, or the like, are also contemplated.

In another embodiment, each wireless medical device **12** includes an optional global positioning module **56** which determines a current geographical position using trilaterization of timing signals receive from global positioning satellites. The global positioning module **56** determines the current geographical location of the wireless medical device **12** and transmits the current geographical location to the location management module **46.** From the current geographical position, the location management module **46** determines the geographical region in which the wireless medical device **12** currently resides.

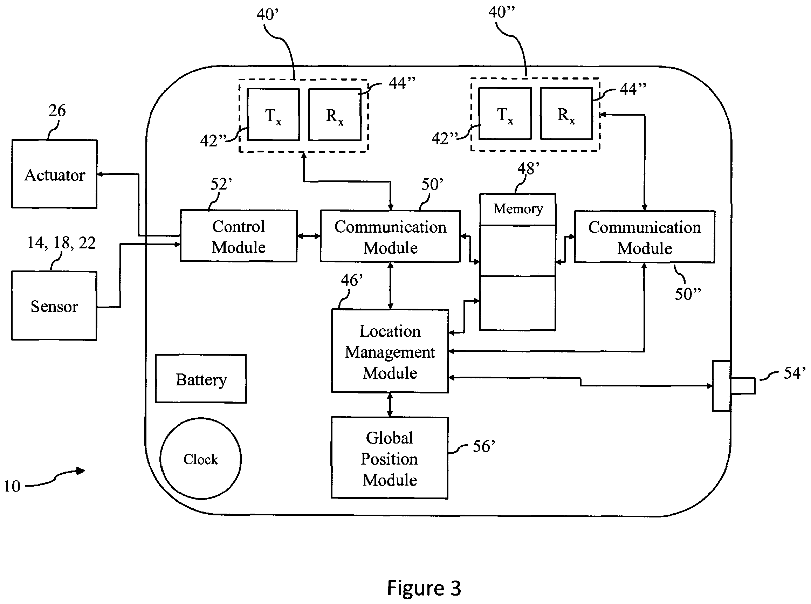

With reference to FIGURE 3, in another embodiment, the wireless medical device wirelessly receives the current geographical location from the hub device **10.** The hub medical device **10** includes a first transceiver **40'** which communicates with the other wireless medical devices of the body network and a second transceiver **40"** which communicates with the infrastructure network **30.** The wireless hub may be connected with a physiological data sensor and/or an actuator like the other wireless medical devices **12,** or may function merely as a central controller or coordinator and for transferring physiological and/or therapy related information to and from the network **30.** Similarly to the wireless medical devices **12,** the hub **10** includes a location management module **46'** which ascertains a current geographical position of the hub **10** and determines an associated geographical region associated with the current geographical position. The location management module **46'** controls the wireless transceivers **40', 40"** to operate according to one of the operating profiles based on the determined geographical region. The location management module **46'** receives the current geographical position from at least one of a user input **54',** global positioning module **56',** and via the infrastructure network **30.** The user input **54'** and global positioning module **56'** function similar to that of the wireless medical devices **12.** Using an input device, such as a switch (as illustrated), button, keyboard, joystick, keypad, touch-screen, touchpad, or other suitable input device, the user can select a geographical region.

As previously described, the hub **10** includes a profile memory **48'** which stores the operating profiles for the wireless medical devices **12** and their transceivers **40** and the transceivers **40', 40"** of hub **10.** Updated operating profiles which reflect changes in regulatory requirements can be obtain wirelessly from the infrastructure network **30** via the transceiver **40".** This is also advantageous if new frequency bands are introduced, for example the proposed MBAN band in the United States. Other changes include frequency band ranges, changes in duty cycle, changes in transmit power, or the like.

Communication modules **50', 50"** receive and transmit information packets to/from the wireless medical devices **12** and the infrastructure network **30,** respectively. The communications module **50'** controls the transceiver **40'** to transmit the packets with an operating profile dictated by the location management module **46'.** Accordingly, The communications module **50"** controls the transceiver **40"** to transmit the packets with an operating profile dictated by the location management module **46'.** If the hub unit **10** is connected with a sensor or actuator, then it also includes a sensor or actuator control module **52'.**

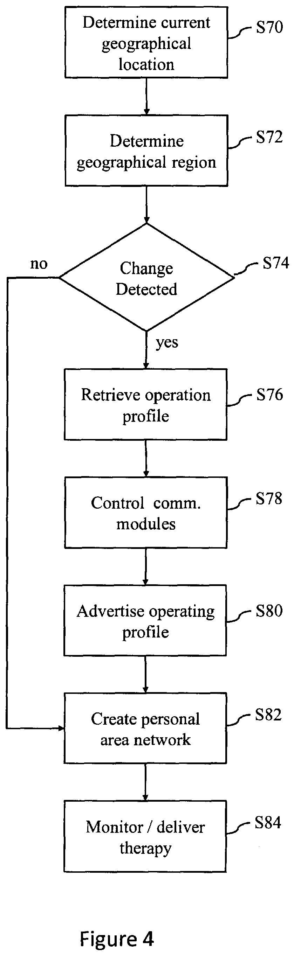

With reference to FIGURE 4, a method for selecting an operating profile and informing other wireless medical devices **12** is illustrated. In one embodiment, the hub device **10** acts as a master device, e.g. in a star network, which advertises the geographical region, current geographical position, or operating profile using information packets or beacon packets. The hub **10** determines the current geographical location **(S70)** from at least one of a user input **54',** global positioning module **56',** and via the infrastructure network **30.** The location management module **46'** determines a geographical region **(S72)** in which the hub **10** and wireless medical devices **12** currently reside according to the determined current geographical location. If a change in the geographical region is detected **(S74),** the location management module **46'** retrieves the operation profile(s) associated with the geographical region **(S76)** from at least one of the profile memory **48'** or from a remote location via the infrastructure network **30.** The location management module **46'** controls the communication modules **50', 50" (S78)** to operate according to the operation profiles retrieved in step **S76.** The communication module **50'** controls the transceiver **40'** to transmit a beacon message to advertise the operating profile **(S80)** to the wireless medical devices **12.** In another embodiment, the operating profile is embedded as part of an information packet, e.g. in a packet/frame transmission the operating profile can be embedded in the MAC address header or the PHY layer, and advertised as such. In a further embodiment, the geographical region or current geographical position rather than the operating profile is advertised to the wireless medical devices **12.** After the wireless medical devices **12** have received the advertised operating profile or geographical region, a personal area network is created **(S82)** and monitoring of physiological data and therapy deliver may ensue **(S84).**

In another embodiment, the devices **10, 12** of the personal area network operate in a peer to peer configuration, e.g. in a mesh network. If one device detects a change in the geographical region which necessitates a change in the operating profile, then the device **10, 12** which detected that change advertises at least one of the geographical region and required operating profile. If the device includes a global position module **56, 56',** then it may also advertise the current geographical position. Upon hearing the advertised mode switch command, the location management module **46, 46'** of the neighboring wireless devices **10, 12** controls the transceivers **40, 40', 40"** accordingly.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A wireless medical device (12), comprising:
at least one of a sensor (14,18,22) configured to monitor physiological data of a patient and an actuator (26) configured to deliver therapy to the patient;
a wireless transceiver (40) configured to transmit and/or receive information packets related to at least one of the monitored physiological data and delivered therapy, the wireless transceiver (40) having a plurality of selectable operating parameters;
**characterised in that** the wireless medical device (12) further comprises a location management module (46) configured to ascertain a current geographical position of the wireless medical device (12), to determine a corresponding geographical region associated with the current geographical position, and to control the wireless transceiver (40) to operate according to one of a plurality of operating profiles defining a plurality of operating parameters for the wireless transceiver associated with regulatory requirements of the determined geographical region;
wherein the location management module (46) is configured to control the wireless transceiver (40) to transmit the determined geographical region to a neighboring wireless medical device (12).

2. The wireless medical device (12) according to claim 1, further including:
a profile memory (48) configured to store the plurality of operating profiles, each operating profile defining a plurality of operating parameters for the wireless transceiver (40) which are associated with at least one geographical region.

3. The wireless medical device (12) according to claim 1, wherein the operating parameters include at least one of frequency, duty cycle, bandwidth and maximum transmit power.

4. The wireless medical device (12) according to either one of claims 1 and 2, further including:
a global positioning module (56) configured to determine the current geographical location of the wireless medical device (12) and to transmit the current geographical location to the location management module (46).

5. The wireless medical device (12) according to any one of claims 1-4, further including:
a user input (54) configured to input the corresponding geographical region to the location management module (46).

6. A wireless patient area network (13), comprising:
a plurality of wireless medical device (12) according to any one of claims 1 -5; and
a wireless hub (10) configured to interface the plurality of wireless medical device (12) to an infrastructure network (30).

7. The wireless patient area network (13) according to claim 6, wherein the wireless hub (10) includes:
a wireless transceiver (40') configured to communicate to the plurality of wireless medical devices (12) to receive the monitored physiological data therefrom and to transmit an actuator control signal to control an actuator to deliver therapy thereto;
location management module (46') which is configured to ascertain a current geographical position of the wireless hub (10) and to control a wireless transceiver (40') to transmit at least one of the current geographical position and the operating profiles for the current geographical position to at least one of the plurality of wireless medical devices (12).

8. The wireless patient area network (13) according to claim 7, wherein the wireless hub (10) further includes:
an infrastructure transceiver (40") configured to communicate with the infrastructure network (30) to transmit and receive information packets related to the wireless medical devices (12).

9. The wireless patient area network (13) according to claim 8, wherein the wireless transceiver (40') and the infrastructure transceiver (40") are the same transceiver.

10. The wireless patient area network (13) according to any one of claims 7-9, wherein the wireless hub (10) further includes:
a global positioning module (56') configured to determine the current geographical location of the wireless hub (10) and to transmit at least one of the current geographical location and the operating profiles for the current geographical position to the location management module (46').

11. The wireless patient area network (13) according to any one of claims 8-10, wherein the location management module (46') is configured to determine the current geographical position from the infrastructure network (30).

12. A method for wirelessly transmitting medical information via a wireless medical device (12), comprising:
monitoring physiological data of a patient;
via a wireless transceiver (40) of the wireless medical device (12), wirelessly transmitting and/or receiving information packets related to at least one of the monitored physiological data and a delivered therapy, the wireless transceiver (40) having a plurality of selectable operating parameters;
and, with a location management module (46) of the wireless medical device (12):
ascertaining a current geographical position of a wireless medical device (12);
determining a corresponding geographical region associated with the current geographical position;
controlling the wireless transceiver (40) to operate according to one of a plurality of operating profiles defining a plurality of operating parameters for the wireless transceiver associated with regulatory requirements of the determined geographical region; and
controlling the wireless transceiver (40) to transmit the determined geographical region to a neighboring wireless medical device (12).

13. The method according to claim 12, further including:
storing the plurality of operating profiles, each operating profile defining a plurality of operating parameters for the wireless transceiver (40) which are associated with at least one geographical region;

14. The method according to either one of claims 12 and 13, further including:
determining the current geographical location of the wireless medical device (12) and transmitting the current geographical location; or
manually determining the corresponding geographical region and transmitting the corresponding geographical region to the location management module (46).

15. The method according to any one of claims 12-14, wherein the operating parameters includes at least one of frequency, duty cycle, bandwidth and maximum transmit power.

## Patentansprüche

1. Drahtlose medizinische Vorrichtung (12), umfassend:
mindestens einen von einem Sensor (14, 18, 22), der zum Überwachen physiologischer Daten eines Patienten konfiguriert ist, und einem Stellglied (26), das zur Abgabe einer Therapie an den Patienten konfiguriert ist;
einen drahtlosen Sender-Empfänger (40), der konfiguriert ist, um Informationspakete zu senden und/oder zu empfangen, die sich auf mindestens eines der überwachten physiologischen Daten und der abgegebenen Therapie beziehen, wobei der drahtlose Sender-Empfänger (40) eine Vielzahl von wählbaren Betriebsparametern aufweist;
**dadurch gekennzeichnet, dass** die drahtlose medizinische Vorrichtung (12) weiter ein Standortverwaltungsmodul (46) umfasst, das konfiguriert ist, um eine aktuelle geografische Position der drahtlosen medizinischen Vorrichtung (12) zu bestimmen, um eine entsprechende geografische Region zu bestimmen, die der aktuellen geografischen Position zugeordnet ist, und um den drahtlosen Sender-Empfänger (40) zu steuern, um gemäß einem von mehreren Betriebsprofilen zu arbeiten, die eine Vielzahl von Betriebsparametern für den drahtlosen Sender-Empfänger definieren, der den gesetzlichen Anforderungen der bestimmten geografischen Region zugeordnet ist;
wobei das Standortverwaltungsmodul (46) konfiguriert ist, um den drahtlosen Sender-Empfänger (40) zu steuern, um den bestimmten geografischen Bereich an eine benachbarte drahtlose medizinische Vorrichtung (12) zu übertragen.

2. Drahtlose medizinische Vorrichtung (12) nach Anspruch 1, weiter beinhaltend:
einen Profilspeicher (48), der konfiguriert ist, um die Vielzahl von Betriebsprofilen zu speichern, wobei jedes Betriebsprofil eine Vielzahl von Betriebsparametern für den drahtlosen Sender-Empfänger (40) definiert, die mindestens einem geografischen Bereich zugeordnet sind.

3. Drahtlose medizinische Vorrichtung (12) nach Anspruch 1, wobei die Betriebsparameter mindestens eines von Frequenz, Tastverhältnis, Bandbreite und maximaler Sendeleistung beinhalten.

4. Drahtlose medizinische Vorrichtung (12) nach einem der Ansprüche 1 und 2, weiter beinhaltend:
ein globales Positionierungsmodul (56), das konfiguriert ist, um den aktuellen geografischen Standort der drahtlosen medizinischen Vorrichtung (12) zu bestimmen und den aktuellen geografischen Standort an das Standortverwaltungsmodul (46) zu übertragen.

5. Drahtlose medizinische Vorrichtung (12) nach einem der Ansprüche 1-4, weiter beinhaltend:
eine Benutzereingabe (54), die konfiguriert ist, um den entsprechenden geografischen Bereich in das Standortverwaltungsmodul (46) einzugeben.

6. Drahtloses Patientenbereichsnetzwerk (13), umfassend:
eine Vielzahl von drahtlosen medizinischen Vorrichtungen (12) nach einem der Ansprüche 1-5; und
einen drahtlosen Hub (10), der konfiguriert ist, um als Schnittstelle zwischen der Vielzahl der drahtlosen medizinischen Vorrichtung (12) und einem Infrastrukturnetzwerk (30) zu fungieren.

7. Drahtloses Patientenbereichsnetzwerk (13) nach Anspruch 6, wobei der drahtlose Hub (10) beinhaltet:
einen drahtlosen Sender-Empfänger (40'), der konfiguriert ist, um mit der Vielzahl von drahtlosen medizinischen Vorrichtungen (12) zu kommunizieren, um die überwachten physiologischen Daten davon zu empfangen und ein Stellgliedsteuersignal zum Steuern eines Stellglieds zur Abgabe einer Therapie dorthin zu übertragen;
Standortverwaltungsmodul (46'), das konfiguriert ist, um eine aktuelle geografische Position des drahtlosen Hubs (10) zu bestimmen und einen drahtlosen Sender-Empfänger (40') zu steuern, um mindestens eine der aktuellen geografischen Positionen und die Betriebsprofile für die aktuelle geografische Position an mindestens eine der Vielzahl von drahtlosen medizinischen Geräten (12) zu übertragen.

8. Drahtloses Patientenbereichsnetzwerk (13) nach Anspruch 7, wobei der drahtlose Hub (10) weiter beinhaltet:
einen Infrastruktur-Sender-Empfänger (40"), der konfiguriert ist, um mit dem Infrastrukturnetzwerk (30) zu kommunizieren, um Informationspakete in Bezug auf die drahtlosen medizinischen Vorrichtungen (12) zu senden und zu empfangen.

9. Drahtloses Patientenbereichsnetzwerk (13) nach Anspruch 8, wobei der drahtlose Sender-Empfänger (40') und der Infrastruktur-Sender-Empfänger (40") derselbe Sender-Empfänger sind.

10. Drahtloses Patientenbereichsnetzwerk (13) nach einem der Ansprüche 7-9, wobei der drahtlose Hub (10) weiter beinhaltet:
ein globales Ortungsmodul (56'), das konfiguriert ist, um den aktuellen geografischen Standort des drahtlosen Hubs (10) zu bestimmen und mindestens einen der aktuellen geografischen Standorte und die Betriebsprofile für die aktuelle geografische Position an das Standortverwaltungsmodul (46') zu übertragen.

11. Drahtloses Patientenbereichsnetzwerk (13) nach einem der Ansprüche 8-10, wobei das Standortverwaltungsmodul (46') konfiguriert ist, um die aktuelle geografische Position aus dem Infrastrukturnetzwerk (30) zu bestimmen.

12. Verfahren zum drahtlosen Übertragen von medizinischen Informationen über eine drahtlose medizinische Vorrichtung (12), umfassend:
Überwachen physiologischer Daten eines Patienten;
über einen drahtlosen Sender-Empfänger (40) der drahtlosen medizinischen Vorrichtung (12), drahtloses Senden und/oder Empfangen von Informationspaketen, die sich auf mindestens eines der überwachten physiologischen Daten und einer abgegebenen Therapie beziehen, wobei der drahtlose Sender-Empfänger (40) eine Vielzahl von wählbaren Betriebsparametern aufweist;
und mit einem Standortverwaltungsmodul (46) der drahtlosen medizinischen Vorrichtung (12):
Bestimmen einer aktuellen geografischen Position einer drahtlosen medizinischen Vorrichtung (12);
Bestimmen einer entsprechenden geografischen Region, die der aktuellen geografischen Position zugeordnet ist;
Steuern des drahtlosen Sender-Empfängers (40), um gemäß einem von mehreren Betriebsprofilen zu arbeiten, die eine Vielzahl von Betriebsparametern für den drahtlosen Sender-Empfänger definieren, die den gesetzlichen Anforderungen des bestimmten geografischen Bereichs zugeordnet sind; und
Steuern des drahtlosen Sender-Empfängers (40), um den bestimmten geografischen Bereich an eine benachbarte drahtlose medizinische Vorrichtung (12) zu übertragen.

13. Verfahren nach Anspruch 12, weiter beinhaltend:
Speichern der Vielzahl von Betriebsprofilen, wobei jedes Betriebsprofil eine Vielzahl von Betriebsparametern für den drahtlosen Sender-Empfänger (40) definiert, die mindestens einem geografischen Bereich zugeordnet sind.

14. Verfahren nach einem der Ansprüche 12 und 13, weiter beinhaltend:
Bestimmen des aktuellen geografischen Standorts der drahtlosen medizinischen Vorrichtung (12) und Übertragen des aktuellen geografischen Standorts; oder
manuelles Bestimmen der entsprechenden geografischen Region und Übertragen der entsprechenden geografischen Region an das Standortverwaltungsmodul (46).

15. Verfahren nach einem der Ansprüche 12-14, wobei die Betriebsparameter mindestens eines von Frequenz, Tastverhältnis, Bandbreite und maximaler Sendeleistung beinhalten.

## Revendications

1. Dispositif médical sans fil (12), comprenant :
au moins l'un d'un capteur (14, 18, 22) configuré pour contrôler des données physiologiques d'un patient et un dispositif d'actionnement (26) configuré pour délivrer une thérapie au patient ;
un émetteur-récepteur sans fil (40) configuré pour émettre et/ou recevoir des paquets d'informations liés à au moins l'une des données physiologiques contrôlées et de la thérapie délivrée, l'émetteur-récepteur sans fil (40) ayant une pluralité de paramètres de fonctionnement pouvant être sélectionnés ;
**caractérisé en ce que** le dispositif médical sans fil (12) comprend en outre
un module de gestion de localisation (46) configuré pour vérifier une position géographique actuelle du dispositif médical sans fil (12), pour déterminer une région géographique correspondante associée à la position géographique actuelle, et pour commander l'émetteur-récepteur sans fil (40) pour qu'il fonctionne selon l'un d'une pluralité de profils de fonctionnement définissant une pluralité de paramètres de fonctionnement pour l'émetteur-récepteur sans fil associés à des exigences réglementaires de la région géographique déterminée ;
dans lequel le module de gestion de localisation (46) est configuré pour commander l'émetteur-récepteur sans fil (40) pour transmettre la région géographique déterminée à un dispositif médical sans fil voisin (12).

2. Dispositif médical sans fil (12) selon la revendication 1, incluant en outre :
une mémoire de profils (48) configurée pour stocker la pluralité de profils de fonctionnement, chaque profil de fonctionnement définissant une pluralité de paramètres de fonctionnement pour l'émetteur-récepteur sans fil (40) qui est associé à au moins une région géographique particulière.

3. Dispositif médical sans fil (12) selon la revendication 1, dans lequel les paramètres de fonctionnement incluent au moins l'un d'une fréquence, d'un rapport cyclique, d'une bande passante et d'une puissance d'émission maximale.

4. Dispositif médical sans fil (12) selon l'une ou l'autre des revendications 1 et 2, incluant en outre :
un module de positionnement global (56) configuré pour déterminer la localisation géographique actuelle du dispositif médical sans fil (12) et pour transmettre la localisation géographique actuelle au module de gestion de localisation (46).

5. Dispositif médical sans fil (12) selon l'une quelconque des revendications 1 à 4, incluant en outre :
une entrée utilisateur (54) configurée pour entrer la région géographique correspondante dans le module de gestion de localisation (46).

6. Réseau local sans fil pour patient (13), comprenant :
une pluralité de dispositifs médicaux sans fil (12) selon l'une quelconque des revendications 1 à 5 ; et
un concentrateur sans fil (10) configuré pour interfacer la pluralité de dispositifs médicaux sans fil (12) à un réseau d'infrastructure (30).

7. Réseau local sans fil pour patient (13) selon la revendication 6, dans lequel le concentrateur sans fil (10) inclut :
un émetteur-récepteur sans fil (40') configuré pour communiquer avec la pluralité de dispositifs médicaux sans fil (12) pour recevoir les données physiologiques contrôlées à partir de ce dernier et pour émettre un signal de commande de dispositif d'actionnement pour commander un dispositif d'actionnement pour délivrer la thérapie à ce dernier ;
un module de gestion de localisation (46') qui est configuré pour vérifier une position géographique actuelle du concentrateur sans fil (10) et pour commander un émetteur-récepteur sans fil (40') pour transmettre au moins l'un de la position géographique actuelle et des profils de fonctionnement pour la position géographique actuelle à au moins l'un de la pluralité de dispositifs médicaux sans fil (12).

8. Réseau local sans fil pour patient (13) selon la revendication 7, dans lequel le concentrateur sans fil (10) inclut en outre :
un émetteur-récepteur d'infrastructure (40") configuré pour communiquer avec le réseau d'infrastructure (30) pour émettre et recevoir des paquets d'informations liés aux dispositifs médicaux sans fil (12).

9. Réseau local sans fil pour patient (13) selon la revendication 8, dans lequel l'émetteur-récepteur sans fil (40') et l'émetteur-récepteur d'infrastructure (40") sont le même émetteur-récepteur.

10. Réseau local sans fil pour patient (13) selon l'une quelconque des revendications 7 à 9, dans lequel le concentrateur sans fil (10) inclut en outre :
un module de positionnement global (56') configuré pour déterminer la localisation géographique actuelle du concentrateur sans fil (10) et pour transmettre au moins l'un de la localisation géographique actuelle et des profils de fonctionnement pour la position géographique actuelle au module de gestion de localisation (46').

11. Réseau local sans fil pour patient (13) selon l'une quelconque des revendications 8 à 10, dans lequel le module de gestion de localisation (46') est configuré pour déterminer la position géographique actuelle à partir du réseau d'infrastructure (30).

12. Procédé pour l'émission sans fil d'informations médicales via un dispositif médical sans fil (12), comprenant :
le contrôle de données physiologiques d'un patient ;
via un émetteur-récepteur sans fil (40) du dispositif médical sans fil (12), l'émission et/ou la réception sans fil de paquets d'informations liés à au moins l'une des données physiologiques contrôlées et d'une thérapie délivrée, l'émetteur-récepteur sans fil (40) ayant une pluralité de paramètres de fonctionnement pouvant être sélectionnés ;
et, avec un module de gestion de localisation (46) du dispositif médical sans fil (12) :
la vérification d'une position géographique actuelle d'un dispositif médical sans fil (12) ;
la détermination d'une région géographique correspondante associée à la position géographique actuelle ;
la commande de l'émetteur-récepteur sans fil (40) pour fonctionner selon l'un d'une pluralité de profils de fonctionnement définissant une pluralité de paramètres de fonctionnement pour l'émetteur-récepteur sans fil associé à des exigences réglementaires de la région géographique déterminée ; et
la commande de l'émetteur-récepteur sans fil (40) pour transmettre la région géographique déterminée à un dispositif médical sans fil voisin (12).

13. Procédé selon la revendication 12, incluant en outre :
le stockage de la pluralité de profils de fonctionnement, chaque profil de fonctionnement définissant une pluralité de paramètres de fonctionnement pour l'émetteur-récepteur sans fil (40) qui sont associés à au moins une région géographique particulière.

14. Procédé selon l'une ou l'autre des revendications 12 et 13, incluant en outre :
la détermination de la localisation géographique actuelle du dispositif médical sans fil (12) et la transmission de la localisation géographique actuelle ; ou
la détermination manuelle de la région géographique correspondante et la transmission de la région géographique correspondante au module de gestion de localisation (46).

15. Procédé selon l'une quelconque des revendications 12 à 14, dans lequel les paramètres de fonctionnement incluent au moins l'un d'une fréquence, d'un rapport cyclique, d'une bande passante et d'une puissance d'émission maximale.
